Europäisches Patentamt

European Patent Office

Office européen des brevets

(19)

(11) **EP 1 095 615 A1**

(12) **EUROPÄISCHE PATENTANMELDUNG**

(43) Veröffentlichungstag:
02.05.2001 Patentblatt 2001/18

(51) Int Cl.⁷: **A61B 5/103, A61B 9/00**

(21) Anmeldenummer: 00810903.5

(22) Anmeldetag: 02.10.2000

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **29.10.1999 EP 99810979**

(71) Anmelder: **Sulzer Biologics Inc.**
**Austin, Texas 78717 (US)**

(72) Erfinder: **Stirnemann, Alfred**
**8702 Zollikon (CH)**

(74) Vertreter: **Sulzer Management AG**
**KS/Patente/0007,**
**Zürcherstrasse 12**
**8401 Winterthur (CH)**

(54) **Vorrichtung und Verfahren zum Erfassen des Zustandes eines Gelenkes**

(57)     Die Vorrichtung (1) zum Erfassen eines Knorpelschadens eines sich innerhalb eines Körpers (5) befindlichen Gelenkes (9) umfasst:

- eine Schwingungserzeugungsvorrichtung (2) zum Erzeugen tieffrequenter Schwingungen, insbesondere im Bereich zwischen 1 und 500 Hz,
- ein Schwingungsübertragungsmittel (3) zum Einleiten der Schwingungen in den Körper (5),
- einen ersten und einen zweiten Sensor (20, 21) zum Messen von Schwingungssignalen des Körpers (5),
- eine Signalverarbeitungsvorrichtung (26d), welche zumindest mit den beiden Sensoren verbunden ist und aus den beiden Sensorsignalen eine mechanische Impedanz ermittelt,
- sowie eine Signalauswertevorrichtung (26e), welche die ermittelte mechanische Impedanz mit vorherbestimmten Faktoren gewichtet und daraus eine Angabe über den Zustand des Gelenkes (9) ermittelt.

Fig.6

EP 1 095 615 A1

**Beschreibung**

Die Erfindung betrifft eine Vorrichtung zum Erfassen des Zustandes, insbesondere des Knorpelzustandes oder des Meniskuszustandes, eines Gelenkes gemäss dem Oberbegriff von Anspruch 1. Die Erfindung betrifft weiter ein Verfahren zum Erfassen des Zustandes eines Gelenkes gemäss dem Oberbegriff der Ansprüche 12 oder 19.

Gelenkschäden sind in der Bevölkerung weit verbreitet und führen mittelfristig zu einer wesentlichen Beeinträchtigung des Wohlbefindens und längerfristig zu einer wesentlichen Beeinträchtigung der Funktion sowie zu erheblichen Schmerzen, weshalb ein stark geschädigtes Gelenk oft durch eine Totalendoprothese zu ersetzen ist. Zur Früherkennung von Gelenkschäden, wie auch zur Voruntersuchung vor einer Operation oder beispielsweise zur postoperativen Kontrolle ist es von entscheidender Bedeutung über eine objektive, nicht invasive Methode zum Erfassen des Knorpelschadens eines Gelenkes, insbesondere des Kniegelenkes zu verfügen.

Bekannt sind arthroskopische Verfahren zum Erfassen eines Knorpelschadens, welche aber den Nachteil eines invasiven Eingriffs in den Körper sowie weitere Komplikationsrisiken aufweisen. Es ist weiter bekannt Knorpelschäden mittels bildgebender Verfahren, insbesondere Kernspintomographie zu ermitteln. Bildgebende Verfahren weisen den Nachteil auf, dass sie die mechanischen Eigenschaften des Gelenkes nicht direkt feststellen können. Diese Verfahren weisen zudem den Nachteil auf, dass sie sehr kosten- und zeitaufwendig und daher für Routineuntersuchungen nicht geeignet sind.

Aus der Druckschrift EP 0 764 842 A2 ist eine Vorrichtung zum Bestimmen der Härte beziehungsweise der Elastizität eines Gewebes bekannt. Diese Vorrichtung umfasst einen mit Ultraschall betriebenen, piezoelektrischen Wandler, welcher auf ein zu untersuchendes Gewebe gepresst wird und in Verbindung mit der Elastizität ("Härte") des Gewebes ein schwingungsfähiges System mit einem Freiheitsgrad bildet. Aus der gemessenen Eigenfrequenz (Resonanzfrequenz) dieses Systems umfassend Wandler, Gewebe usw., können Rückschlüsse auf die Härte bzw. Elastizität des Gewebes gezogen werden. Nachteilig an dieser bekannten Vorrichtung ist die Tatsache, dass damit nur die lokale Härte des Gewebes am Ort der Schwingungseinleitung messbar ist, weshalb aus den derart ermittelten Werten keine Aussage über den Zustand eines komplexen, mehrere Freiheitsgrade aufweisende Systems wie eines Gelenkes möglich ist. Als weiterer Nachteil ist die Tatsache zu erachten, dass, auf Grund der nur lokalen Messmöglichkeit, zur Untersuchung von sich innerhalb des Körpers befindlichem Gewebe nur ein arthroskopisches Vorgehen möglich ist, indem die Haut eröffnet wird und die Vorrichtung in den Körper eingeführt wird, damit die Vorrichtung das zu untersuchende Gewebe direkt anregen und die Resonanzfrequenz messen kann.

Der vorliegenden Erfindung liegt daher die Aufgabe zugrunde, eine Vorrichtung sowie ein Verfahren zum Erfassen des Zustandes eines Gelenkes zu bilden, die einfach anwendbar und kostengünstig ist.

Diese Aufgabe wird gelöst mit einer Vorrichtung aufweisend die Merkmale von Anspruch 1. Die Unteransprüche 2 bis 11 betreffen weitere, vorteilhaft ausgestaltete Weiterbildungen. Die Aufgabe wird weiter gelöst mit einem Verfahren aufweisend die Merkmale der Ansprüche 12 oder 19. Die Unteransprüche 13 bis 18 betreffen weitere, vorteilhafte Verfahrensschritte.

Die Aufgabe wird insbesondere gelöst mit einer Vorrichtung zum Erfassen des Zustandes eines sich innerhalb eines Körpers bzw. eines Organismus befindlichen Gelenkes, insbesondere zum Erfassen von Schädigungen und/ oder degenerativen Veränderungen des Knorpels, des Meniskus und/oder der Bänder, umfassend eine Schwingungserzeugungsvorrichtung zum Erzeugen tieffrequenter Schwingungen, insbesondere im Bereich zwischen 1 und 500 Hz, ein Schwingungsübertragungsmittel zum Einleiten der Schwingungen in den Körper, einen ersten und einen zweiten Sensor zum Messen von Schwingungssignalen des Körpers, eine Signalverarbeitungsvorrichtung, welche zumindest mit den Sensoren verbunden ist und aus den Sensorsignalen Werte der mechanischen Impedanz bei zumindest zwei unterschiedlichen Frequenzen (Erregerfrequenzen oder Anregungsfrequenzen) ermittelt, sowie eine Signalauswertevorrichtung, welche aus der ermittelten mechanischen Impedanz und vorherbestimmten Parametern mit Hilfe einer Ergebnisfunktion eine Ergebnisgrösse ermittelt, welche den Zustand des Gelenkes objektiv bewertet beziehungsweise beschreibt.

Die Aufgabe wird weiter insbesondere gelöst mit einem Verfahren zum Erfassen des mechanischen Zustandes, insbesondere des Knorpel- oder Meniskuszustandes, eines sich innerhalb des Körpers befindlichen Gelenkes, wobei in einem ersten Verfahrensschritt eine Trainings- oder Lernphase durchgeführt wird,

- indem eine Mehrzahl von Gelenken unterschiedlicher Personen einem Schwingungsanregungssignal ausgesetzt wird, die resultierende Körperschwingung gemessen und daraus die mechanische Impedanz ermittelt wird,
- indem der Zustand der Gelenke klassifiziert wird,
- und indem zwischen den mechanischen Impedanzen und den Zuständen der Gelenke ein mathematischer Zusammenhang wie beispielsweise eine Korrelation ermittelt wird,
  und wobei in einem zweiten Verfahrensschritt eine Bewertung durchgeführt wird,
- indem ein zu untersuchendes Gelenk dem Schwingungsanregungssignal ausgesetzt wird,
- indem die resultierende Körperschwingung gemessen und daraus die mechanische Impedanz ermittelt wird,

- und indem aus der mechanischen Impedanz mit Hilfe des im ersten Verfahrensschritt ermittelten mathematischen Zusammenhangs der Zustand des Gelenkes bewertet wird.

Der mathematische Zusammenhang kann beispielsweise durch eine Ergebnisfunktion beschrieben werden, welche von Parametern und Eingangsvariablen abhängt. Die gemessenen Impedanzwerte bilden die Eingangsvariablen. Die Ergebnisfunktion erlaubt somit ausgehend von der mechanischen Impedanz eine Ergebnisgrösse zu berechnen, deren Wert den Zustand des Gelenkes beschreibt. Dieser Wert ist das Ergebnis einer quantitativen Messung, was gegenüber dem bisher bekannten, auf Beobachtungen basierenden Methoden den Vorteil aufweist, dass diese quantitative Messung objektive Werte ergibt.

Die Ergebnisgrösse e kann beispielsweise wie folgt als lineare Ergebnisfunktion definiert sein:

$$e = \sum_n g_n z_n = g_1 z_1 + g_2 z_2 + \ldots g_n z_n$$

Wobei die Faktoren z ermittelte, diskrete Werte der mechanischen Impedanz darstellen, und die Faktoren g die in der Trainingsphase ermittelten Parameter darstellen. Sobald die Faktoren g bekannt sind kann die Ergebnisgrösse e mit Hilfe der Ergebnisfunktion berechnet werden, indem die diskreten Werte der gemessenen, mechanischen Impedanz mit den Faktoren g gewichtet werden. Die mechanische Impedanz kann als Amplitude, Phase oder als komplexe Grösse berücksichtigt werden.

Die Ergebnisgrösse e kann beispielsweise auch wie folgt als allgemeine nichtlineare Ergebnisfunktion definiert sein:

$$e = f(z_1, \ldots, z_n ; g_1, \ldots g_m)$$

Wobei die Ergebnisgrösse e als nichtlineare Funktion f der Parameter g sowie der Eingangsvariablen z (Impedanzwerte) resultiert.

Die Ergebnisfunktion kann derart definiert sein, dass noch weitere Parameter einen Einfluss auf die Ergebnisgrösse ausüben, beispielsweise das Alter, die Körpergrösse, das Körpergewicht oder das Geschlecht der untersuchten Person. Die Ergebnisfunktion kann auch derart definiert sein, dass sie mehr als eine Ergebnisgrösse liefert.

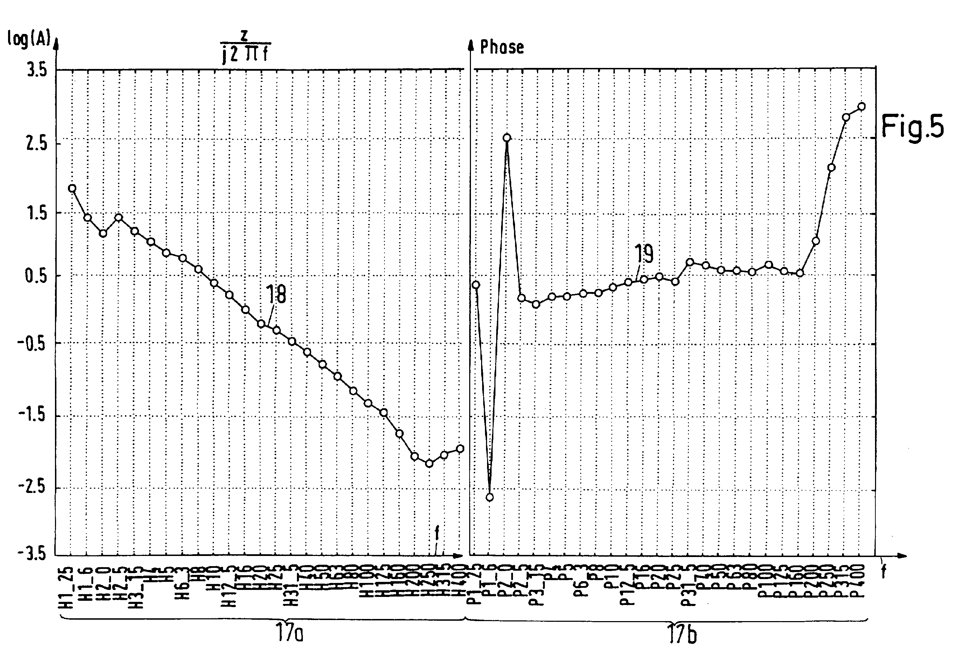

Die dem erfindungsgemässen Verfahren zugrunde liegende Erkenntnis basiert darauf, dass das Gelenk, beispielsweise das Kniegelenk, als ein mechanisches System bestehend aus mehreren idealen Elementen, daher einer Kombination von Federn, Massen und Dämpfern, beschrieben werden kann. Die charakteristischen Eigenschaften dieses mechanischen Systems lassen sich dadurch, dass das mechanische System in Schwingung versetzt wird, messen, wobei es zur Beschreibung eines Systems mit mehreren Freiheitsgraden erforderlich ist das mechanische System mit unterschiedlichen Frequenzen anzuregen. Aus entsprechend gemessenen Werten kann die mechanische Impedanz des mechanischen Systems berechnet beziehungsweise identifiziert werden. Unter dem Begriff mechanische Impedanz wird nachfolgend die Kurve der komplexen, mechanischen Impedanz bzw. Schwingungsantwort in Funktion der Frequenz verstanden, wobei diese mechanische Impedanz, wie in der nachfolgenden Fig. 5 dargestellt, eine Amplitude in Funktion der Frequenz sowie eine Phase in Funktion der Frequenz umfasst. Um den Verlauf dieser mechanischen Impedanz anzunähern müssen deren Werte bei zumindest zwei unterschiedlichen Frequenzen bekannt sind. Vorzugsweise werden deren Werte bei einer Mehrzahl unterschiedlicher Frequenzen ermittelt, um einen genauen Verlauf der mechanischen Impedanz zu erhalten, beziehungsweise um damit die mechanischen Eigenschaften des zu untersuchenden, mehrere Freiheitsgrade aufweisenden Gelenks zu identifizieren. Ein Schaden des Knorpels, des Meniskus oder der Bänder im Kniegelenk bewirkt eine Veränderung des mechanischen Systems, was eine Änderung der mechanischen Impedanz zur Folge hat. Daher ist es möglich durch Messen der mechanischen Impedanz des Gelenks dessen mechanische Eigenschaften zu messen und daraus einen Hinweis auf vorhandene Knorpelschäden zu erhalten. Die mechanische Impedanz wird gemessen, indem der vibrierende Teil einer Schwingungserzeugungsvorrichtung in der Nähe des zu untersuchenden Gelenkes an die Körperoberfläche angepresst wird, sodass die erzeugten Schwingungen, ausgehend von der Körperoberfläche über die Haut und das Muskelgewebe, das Gelenk zu Schwingungen anregen. Vorzugsweise wird der vibrierende Teil an einer derartigen Stelle an der Körperoberfläche angesetzt, dass sich zwischen der Körperoberfläche und dem Gelenk eine nur dünne Schicht Weichteile befindet, sodass die Vibration möglichst gut in den Knochen des Gelenkes eingeleitet wird. In einer möglichen Ausführungsform wird der der Anregungsstelle gegenüberliegende Teil des Gelenks mechanisch fixiert oder eingespannt oder mit zusätzlichen Massen versehen.

Als Schwingungsanregungssignal ist vorzugsweise eine tieffrequente Schwingung, insbesondere mit einer Bandbreite zwischen 1 und 500 Hz geeignet. Wird das Gelenk mit einer derart tieffrequenten, periodischen Kraft belastet, so erfährt das Gelenk eine zeitliche variierende elastische Deformation, wobei das Ausmass der Deformation von mechanischen Eigenschaften des Gelenks abhängt. Durch die Messung der mechanischen Impedanz lassen sich daher Rückschlüsse auf den Zustand des Knorpels ziehen.

Die mechanische Impedanz wird üblicherweise definiert als das Verhältnis der komplexen Amplitude der Kraft zur komplexen Amplitude der Geschwindigkeit für einfache, sinusförmige Bewegungen im eingeschwungenen Zustand. Es gilt somit:

$$Z = F / v$$

Wobei Z = mechanische Impedanz, F = Kraft und v = Geschwindigkeit bedeutet. In der vorliegenden Offenbarung werden unter dem Begriff "mechanische Impedanz" auch abgeleitete Grössen verstanden, wie beispielsweise der Quotient F/a (a = Beschleunigung), F/x (x = Weg) oder auch die Reziprokwerte davon bzw. Kraft / Weggrösse. Die mechanische Impedanz des Gelenks ist beispielsweise messbar, indem als Schwingungsanregungssignal ein sinusförmiges Signal verwendet wird, dessen Frequenz langsam erhöht oder verringert wird, bis die interessierende Bandbreite von beispielsweise 2 bis 400 Hz überstrichen ist. Ein derartiges Schwingungsanregungssignal wird auch als ein Sweep-Signal bezeichnet. Die Frequenz wird vorzugsweise derart langsam erhöht oder verringert, dass sich das angeregte System bei jeder Frequenz mit hinreichender Genauigkeit in einem quasi eingeschwungenen Zustand befindet.

Zum Messen der Kraft F sowie der Geschwindigkeit v werden an sich bekannte Messeinrichtungen verwendet, welche beispielsweise beide am gegen den Körper hin ausgerichteten Endabschnitt des vibrierenden Teils der Schwingungserzeugungsvorrichtung angeordnet sind. Als Messeinrichtungen sind insbesondere Wandlerelemente aus piezoelektrischem Material geeignet, beispielsweise Kraft- und Beschleunigungsaufnehmer. Dem als Beschleunigungsaufnehmer ausgestaltetem Wandlerelement kann ein Ladungsverstärker oder ein separater Vorverstärker nachgeschaltet sein, welcher mit einem Integrator verbunden ist, sodass ebenso die Geschwindigkeit oder die Amplitude der Schwingung messbar ist. Die gemessenen Signale werden einer Signalverarbeitungsvorrichtung zugeführt, welche erlaubt die Amplitude und die Phase der mechanischen Impedanz in Funktion der Anregungsfrequenz zu berechnen. Als Schwingungserzeugungsvorrichtung ist beispielsweise ein elektrodynamischer Vibrator geeignet.

Das erfindungsgemässe Verfahren umfasst in einem ersten Verfahrensschritt eine Trainingsphase, während welcher die mechanische Impedanz in Funktion der Frequenz von Gelenken, insbesondere Kniegelenken unterschiedlicher Personen gemessen wird. Die Trainingsphase kann auch als eine Lernphase oder eine Eichung bezeichnet werden. Zudem können weitere Daten, wie das Alter oder das Gewicht oder das Geschlecht der Person erfasst werden. Nebst der Impedanz wird auch der Zustand der Gelenke erfasst, indem deren Schädigung nach klinisch und/oder biologisch und/oder biochemisch und/oder histologisch definierten Kriterien erfasst werden. Nebst eindeutig gesunden Kniegelenken werden kranke Kniegelenke beispielsweise derart erfasst, dass die Impedanz eines Kniegelenks vor einer Operation gemessen wird, und in einer nachfolgenden Operation der Knorpelschaden des Gelenks gemäss den definierten Kriterien erfasst wird. Somit steht eine Sammlung von Werten zur Verfügung, welche jeweils die mechanische Impedanz in Funktion der Frequenz sowie den nach definierten Kriterien erfasste Zustand des Knorpelschadens umfasst, gegebenenfalls mit Zusatzdaten wie klinischer Befund, Alter, Geschlecht oder Gewicht. Zum Abschluss der Trainingsphase wird zwischen diesen mechanischen Impedanzen, gegebenenfalls unter Verwendung der Zusatzdaten, und den Zuständen des Knorpelschadens ein mathematischer Zusammenhang ermittelt, beispielsweise mittels einer Diskriminanzanalyse. Die dadurch ermittelten, relevanten Parameter der Ergebnisfunktion werden in einer Signalauswertevorrichtung gespeichert. Im zweiten Verfahrensschritt wird der Zustand des Knorpels eines Gelenks erfasst, indem die mechanische Impedanz des zu untersuchenden Gelenks gemessen wird und aus der gemessenen mechanischen Impedanz mit Hilfe des ermittelten mathematischen Zusammenhangs der Zustand des Gelenks bewertet beziehungsweise klassifiziert wird. Es wird daher mit Hilfe der Ergebnisfunktion und der gemessenen mechanischen Impedanz die Ergebnisgrösse berechnet. Sobald die Werte der mechanischen Impedanz vorliegen kann mit relativ wenigen Rechenoperationen der Zustand des Gelenkes klassifiziert werden, sodass als Resultat der Untersuchung innerhalb weniger Sekunden ein Ergebnis vorliegt, welches einen Arzt bei der Erstellung einer medizinischen Diagnose unterstützen kann.

Die erfindungsgemässe Vorrichtung beziehungsweise das erfindungsgemässe Verfahren weist die Vorteile auf, dass sich der Knorpelzustand eines Gelenkes nicht invasiv, ohne Nebenwirkungen, rasch, billig und mit guter Reproduzierbarkeit erfassen lässt. Die erfindungsgemässe Vorrichtung weist eine einfache Bedienung auf, ist leicht, robust, wenig störungsanfällig, transportierbar, und erlaubt einem Arzt zudem eine schnelle Diagnose. Die erfindungsgemässe Messmethode, auch als Impedanzarthrographie bezeichnet, ermöglicht es erstmals, die Gelenke einer breiten Bevölkerungsschicht bezüglich Knorpelschäden zu untersuchen, was eine frühzeitige Erkennung von Schäden erlaubt. Wird die Knorpelschädigung in einem frühzeitigen Stadium erkannt, so kann der Knorpeldefekt beispielsweise mit einer biologischen Reparatur oder einem Implantat geheilt oder zumindest reduziert werden. Die erfindungsgemässe Vorrichtung ist natürlich auch geeignet bekannte und teure Vorrichtungen basierend auf der Methode der Arthroskopie oder der Kernspintomographie zu ersetzen, um beispielsweise Voruntersuchungen, postoperative Kontrollen oder ambulante Therapiekontrollen durchzuführen. Die erfindungsgemässe Vorrichtung ist auch für kostengünstige Voruntersuchungen geeignet, sodass nur bei einem begründeten Hinweis auf einen Knorpelschaden nachfolgend aufwendigere Diagnosemethoden durchzuführen sind. Dies ermöglicht kostenaufwendige Diagnoseverfahren nur dann durchzuführen, wenn sie medizinisch begründet sind, was erhebliche Kosten spart.

Die Schwingungserzeugungsvorrichtung kann als elektromechanischer, elektromagnetischer oder piezoelektrischer Wandler, beispielsweise als elektrodynamischer Vibrator ausgestaltet sein, oder als mechanisch oder fluiddynamisch angeregte Vorrichtung, beispielsweise als hydrodynamischer Vibrator. Ebenso könnte die Schwingungserzeugungsvorrichtung als einen Schlag bewirkendes Instrument, beispielsweise als ein Hammer ausgestaltet sein.

Als Schwingungsanregungssignal ist insbesondere ein Sinussignal, ein Rauschen oder ein Impulssignal, d. h. ein Schlag, geeignet. Als Anregungssignal kann beispielsweise ein Sinussignal mit konstanter oder langsam variierender Frequenz oder ein "chirp"-Signal, d.h. ein Sinussignal welches durch ein definiertes Frequenzband läuft, verwendet werden.

Nebst dem Kniegelenk kann mit der erfindungsgemässen Vorrichtung auch der mechanische Zustand weiterer Gelenke, beispielsweise des Hüftgelenks, des Schultergelenks oder der Wirbelsäule untersucht werden. Die erfindungsgemässe Vorrichtung eignet sich zur Untersuchung der Gelenke von Säugern, insbesondere des Menschen.

Das erfindungsgemässe Verfahren verwendet zur Schwingungsanregung tiefe Frequenzen im Bereich zwischen 1 Hz und 500 Hz. Dies erlaubt, den grössten Teil des durch die Schwingung erzeugten Kraftflusses in das Gelenk zu leiten und so die mechanische Impedanz des Gelenkes zu messen. Im Gegensatz dazu würde die Verwendung von Ultraschall eine undefinierte Wellenausbreitung im Gewebe bewirken und eine Energiedissipation in den Weichteile erfolgen, sodass die Knochen nicht zur Schwingung angeregt würden und somit die mechanische Impedanz des Gelenkes nicht messbar wäre. Zur Schwingungsanregung sind Frequenzen von insbesondere zwischen 1 Hz und 5000 Hz geeignet.

Die Erfindung wird nachfolgend an Hand mehrerer Ausführungsbeispiele im Detail beschrieben. Es zeigen:

Fig. 1                          eine Aufsicht auf das Kniegelenk mit einem auf die mediale Femurkondyle wirkenden Vibrator;

Fig. 2                          ein mechanisches Ersatzschaltbild der Anordnung gemäss Fig. 1;

Fig. 3a, 3b, 3c, 3d             unterschiedliche Anordnungen der Sensoren zur Schwingungserfassung sowie unterschiedliche Einspannungen des Knies;

Fig. 4                          bevorzugte Schwingungseinleitpunkte am Knie;

Fig. 5                          Amplituden- und Phasengang einer gemessenen mechanischen Impedanz;

Fig. 6                          den schematischen Aufbau einer Diagnosevorrichtung;

Fig. 7                          den schematischen Aufbau einer weiteren Diagnosevorrichtung;

Fig. 8                          ein Verfahren zum Auswerten der Messdaten;

Fig. 9                          eine Ausgestaltung einer Diagnosevorrichtung.

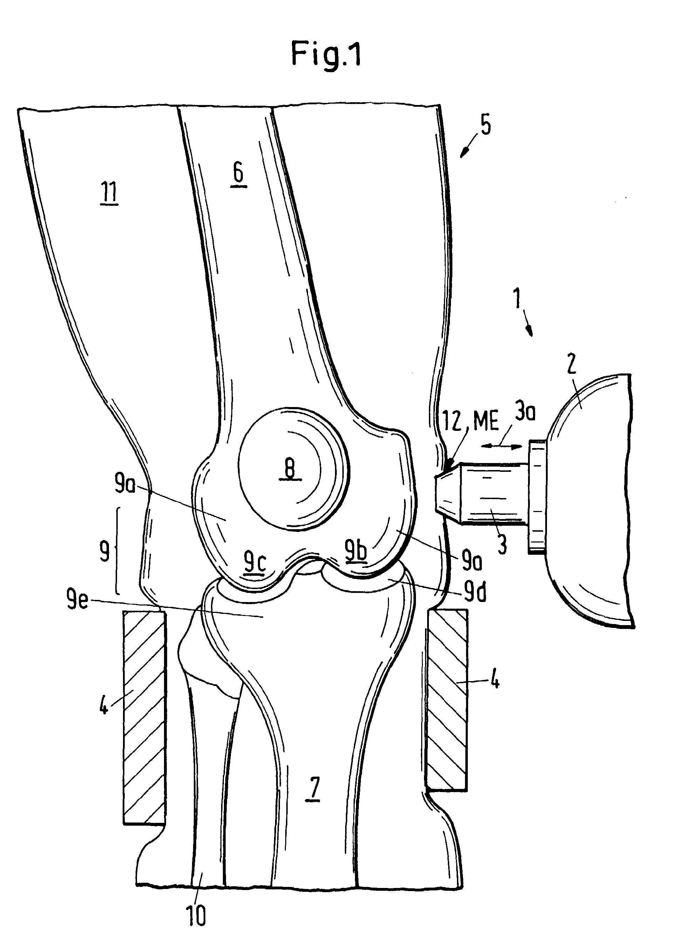

Das in Fig. 1 schematisch dargestellte Körperteil 5 zeigt einen Teilausschnitt des rechten Beines eines Menschen. Das Femur 6, die Tibia 7 sowie die Fibula 10 sind über das Gelenk 9 beweglich miteinander verbunden. Das Gelenk 9 umfasst ein erstes Gelenkteil 9a mit einer medialen Kondyle 9b sowie einer lateralen Kondyle 9c, sowie ein zweites Gelenkteil 9e, sowie ein zwischen den beiden Gelenkteilen 9a, 9e angeordnetes Zwischenteil 9f, welches auch den Meniskus 9d umfasst. Vor dem ersten Gelenkteil 9a ist die Patella 8 angeordnet. Im wesentlichen sind zumindest die sich gegenseitig berührenden Flächen des ersten und zweiten Gelenkteils 9a, 9e mit einer Knorpelschicht belegt. Diese Knorpelschicht ist für die Beweglichkeit des Knies von zentraler Bedeutung. Degenerative Vorgänge oder eine starke Beanspruchung des Knies führen zu einer Schädigung der Knorperschicht was zu einer erheblichen Beeinträchtigung der Beweglichkeit des Knies sowie zu starken Schmerzen führt.

Aus Fig. 1 ist ein Teil der Vorrichtung 1 zum Erfassen eines Knorpelschadens erkennbar, nämlich eine Schwingungserzeugungsvorrichtung 2 sowie ein daran angekoppeltes Schwingungsübertragungsmittel 3, welches eine in Bewegungsrichtung 3a verlaufende Schwingung in den Körper 5 einleitet. An der Oberfläche des Körpers 5 wird ein Ankoppelungsbereich 12 definiert, über welchen die Schwingung in den Körper 5 eingeleitet wird. Der Ort des Ankoppelungsbereiches 12 wird vorzugsweise derart gewählt, dass sich zwischen dem Ankoppelungsbereich 12 und dem anzuregenden Knochen 6,9 möglichst wenig Weichteilgewebe 11 befindet. In der dargestellten Anordnung wird die Schwingung über die Epicondylus medialis (ME) in das erste Gelenkteil 9a eingeleitet. Das zweite Gelenkteil 9e ist in einer Haltevorrichtung 4 eingespannt und darin fest gehalten.

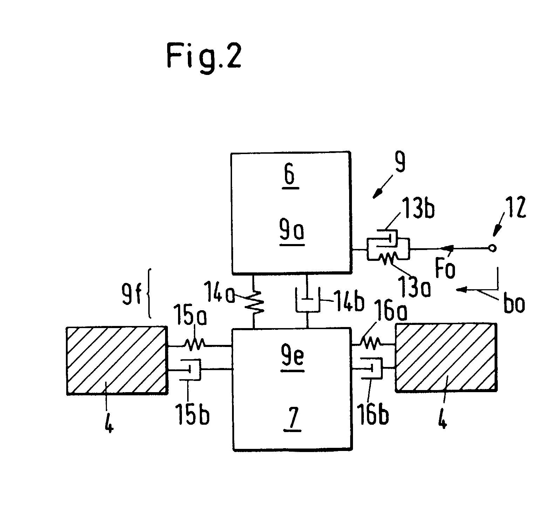

Fig. 2 zeigt das mechanische Ersatzschaltbild der Anordnung gemäss Fig. 1. Die schematisch als Quadrat dargestellten Knochenteile Femur 6 mit erstem Gelenkteil 9a sowie Tibia 7 mit zweitem Gelenkteil 9e sind über das Zwischenteil 9f mechanisch gekoppelt, wobei das Zwischenteil 9f als eine Feder 14a mit Dämpfung 14b nachgebildet ist. Die Schwingungseinleitung mit Kraft Fo und Beschleunigung bo erfolgt über den Ankoppelungsbereich 12, welcher über eine Feder 13a sowie eine Dämpfung 13b an das erste Gelenkteil 9a gekoppelt ist. Das zweite Gelenkteil 9e ist über Federn 15a, 16a und Dämpfungen 15b, 16b an die Einspannvorrichtung 4 gekoppelt. Am Schwingungsübertragungsmittel 3 sind Sensoren zum Erfassen der Kraft Fo sowie der Beschleunigung bo angeordnet, sodass mit der dargestellten Anordnung die mechanische Eingangsimpedanz des Gelenkes 9 messbar ist.

Von besonderem Interesse ist die mechanische Impedanz des Gelenkes 9 umfassend das Zwischenteil 9f sowie die beiden Gelenkteile 9a, 9e, wogegen die Schwingungseinleitung in das erste Gelenkteil 9a sowie die Einspannung des zweiten Gelenkteils 9e in die Haltevorrichtung 4 derart zu erfolgen hat, dass sich die Federn 13a, 15a, 16a und Dämpfungen 13b, 15b, 16b nur gering auf den Wert der mechanischen Eingangsimpedanz auswirken.

Sofern das Ansteuerungssignal der Schwingungserzeugungsvorrichtung bekannt ist, genügt es eine einzige Systemschwingung zu messen, beispielsweise die Kraft Fo oder die Beschleunigung bo, da in diesem Fall unter Verwendung des Ansteuerungssignales sowie der gemessenen Systemschwingung eine der Impedanz ähnliche Grösse berechenbar ist. Diese Schwingungserzeugungsvorrichtung weist vorzugsweise die Eigenschaft auf, dass sich durch die Ankoppelung an den Körper die Kraft bzw. der Verschiebungsweg nicht oder nur sehr gering verändert gegenüber dem nicht angekoppelten Verhalten, weshalb die Schwingungserzeugungsvorrichtung in einer möglichen Ausführungform als eine im wesentlichen ideale Kraftquelle oder eine im wesentlichen ideale Verschiebungsquelle ausgestaltet ist. .

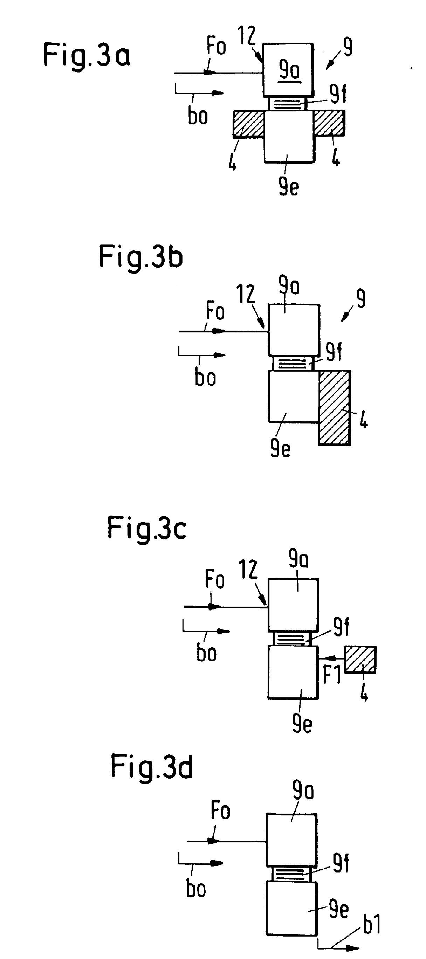

Die Figuren 3a bis 3d zeigen schematisch verschiedene Anordnungen zur Impedanzmessung. Die Anordnung gemäss Fig. 3a zeigt die in Fig. 2 dargestellte Anordnung in nochmals vereinfachter Darstellung, indem die Federn 13a, 15a, 16a und Dämpfungen 13b, 15b, 16b der Einfachheit halber nicht dargestellt sind. In der Anordnung gemäss Fig. 3b ist das Gelenk 9 einseitig an die flächig ausgestaltete Abstützvorrichtung 4 angelehnt. Die Schwingungseinleitung erfolgt über den Ankoppelungsbereich 12 und die Kraft Fo sowie die Beschleunigung bo wird ebenfalls an dieser Stelle gemessen, um die mechanische Eingangsimpedanz zu bestimmen. In der Anordnung gemäss Fig. 3c ist zwischen der Abstützvorrichtung 4 und dem zweiten Gelenkteil 9e eine Kraftmessvorrichtung angeordnet, um die Reaktionskraft F1 zu messen. Diese Anordnung erlaubt mit Hilfe der gemessenen Beschleunigung bo und der Kräfte Fo, F1 die Kraftübertragungsfunktion sowie die Übertragungsimpedanz zu bestimmen. Die Kraftübertragungsfunktion liefert zusätzlich Information bezüglich den Systemeigenschaften und kann beispielsweise hilfreich sein zur mechanischen Parameteridentifikation. In der Anordnung gemäss Fig. 3d wird keine Abstützvorrichtung 4 verwendet. Die Schwingungseinleitung erfolgt wiederum über den Ankoppelungsbereich 12. Die mechanische Eingangsimpedanz ist über die Kraft Fo sowie die Beschleunigung bo messbar. Zusätzlich kann am zweiten Gelenkteil 9e ein Beschleunigungssensor angebracht werden, um die Beschleunigung b1 zu messen, sodass mit den Beschleunigungswerten bo und b1 die mechanische Beschleunigungsübertragungsfunktion bestimmbar ist. Der Ankoppelungsbereich 12 kann natürlich auch im Bereich des zweiten Gelenkteils 9e gewählt werden.

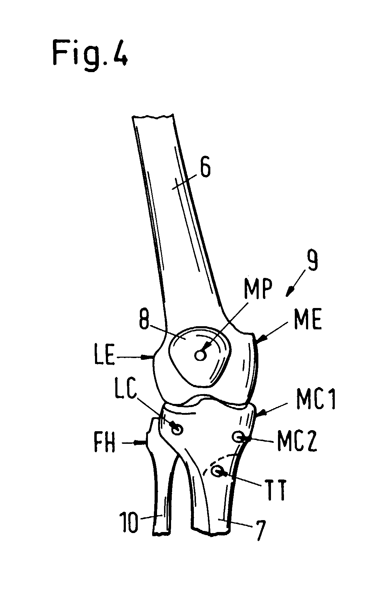

Fig. 4 zeigt eine Ansicht des Kniegelenkes 9 mit bevorzugten Ankoppelungsbereichen 12, welche sich dadurch auszeichnen, dass sich zwischen dem Ankoppelungsbereich 12 und dem anzuregenden Knochen wenig Haut bzw. wenig Weichteilgewebe 11 befindet. Die bevorzugten Ankoppelungsbereiche 12 sind das Zentrum der Patella MP, die Epicondylus medialis ME oder lateralis LE, die Condylus medialis MC1, MC2 oder lateralis LC, die Tuberositas tibiae TT oder die Tibiofibularis FH.

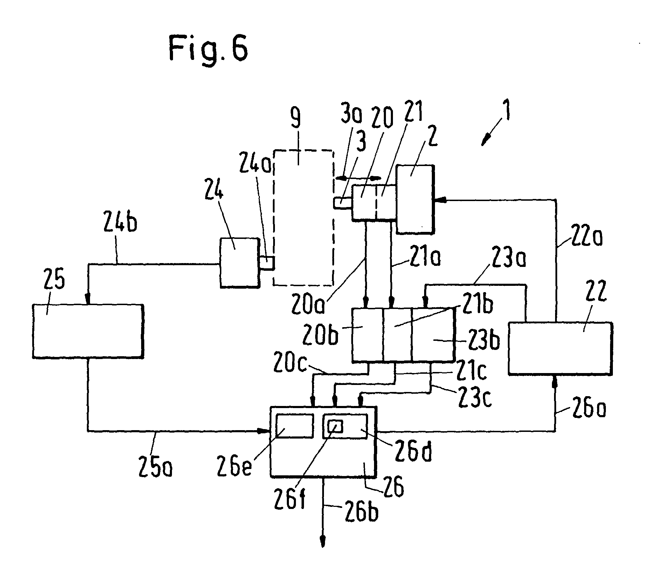

Fig. 6 zeigt schematisch den Aufbau einer Vorrichtung 1 zum Erfassen eines Knorpelschadens. Die elektromagnetisch angetriebene Schwingungserzeugungsvorrichtung 2 ist über ein Schwingungsübertragungsmittel 3 und unter Ausübung einer bestimmten Anpresskraft an das Gelenk 9 angekoppelt, um eine in Richtung 3a wirkende Schwingung in das Gelenk 9 einzuleiten. An der Schwingungserzeugungsvorrichtung 2 beziehungsweise am Schwingungsübertragungsmittel 3 ist ein erster Sensor 20 zur Kraftmessung sowie ein zweiter Sensor 21 zur Beschleunigungsmessung angeordnet. Ein Signalgenerator 22 ist über eine elektrische Leitung 22a mit der als Vibrator ausgestalteten Schwingungserzeugungsvorrichtung 2 verbunden.

Als Sensor 20 zur Kraftmessung ist beispielsweise der Sensortyp B&K 8200, als Sensor 21 zur Beschleunigungsmessung der Sensortyp B&K 4366 und als elektrodynamischer Vibrator der Shakertyp B&K 4810 oder B&K 4811 geeignet, alles Geräte der Firma Brüel & Kjaer, Dänemark. Der elektrodynamische Vibrator weist vorzugsweise eine Federsteifigkeit zwischen 50 N/cm und 150 N/cm auf.

Die Anordnung gemäss Fig. 6 erlaubt ein Schwingungssignal mit einem Frequenzbereich beginnend zwischen 1 und 4 Hz bis 400 Hz zu erzeugen. Zum sicheren und reproduzierbaren Einleiten der Schwingung in des Gelenk 9 wird der Vibrator 2 mit einer Vorspannungskraft von 15 N bis 30 N gegen den Körper 5 gedrückt. Der Signalgenerator 22 ist über eine Leitung 26a mit einem Computer 26 verbunden und wird von diesem angesteuert. Das elektrische Schwingungssignal des Signalgenerators 22 wird über eine elektrische Leitung 23a einem Vorverstärker 23b und daraufhin über eine Leitung 23c dem Computer 26 zugeführt. Ebenso wird das Signal der beiden Sensoren 20, 21 über Leitungen 20a, 21a einem Verstärker 20b, 21b zugeführt und nachfolgend über Leitungen 20c, 21c dem Computer 26 zugeführt. Der Computer 26 ist über eine Ein-/Ausgabeleitung 26b mit einer Ein- und Ausgabevorrichtung verbunden. Der Computer 26 umfasst eine Signalverarbeitungsvorrichtung 26d, welche aus den gemessenen Werten der beiden Sensoren 20, 21 die mechanische Impedanz zu berechnen erlaubt. Die Signalverarbeitungsvorrichtung 26d umfasst zudem einen Terzbandanalysator 26f, um aus den Signalen der Sensoren 20, 21 gemittelte Terzbandspektren zu erzeugen, und um daraus nachfolgend die diskreten Impedanzwerte zu berechnen.

In einer weiteren Ausgestaltung könnte die Vorrichtung 1 zudem einen weiteren Sensor 24 mit Ankoppelungsmittel 24a umfassen, welcher über eine Leitung 24b mit einem Verstärker und über eine nachfolgende Leitung 25a mit dem Computer 26 verbunden ist. Der Sensor 24 ist als ein Beschleunigungssensor oder als ein Kraftsensor ausgestaltet.

Das erfindungsgemässe Verfahren zum Erfassen eines Knorpelschadens umfasst zwei Verfahrensschritte, eine Trainingsphase und eine Diagnosephase. Während der Trainingsphase wird die mechanische Impedanz einer Vielzahl von Gelenken unterschiedlicher Personen mit der Vorrichtung 1 gemessen. Hierzu wird das Schwingungsübertragungsmittel 3 an das Gelenk 9 angelegt, und daraufhin mit Hilfe des Signalgenerators 22 ein durchlaufendes, frequenzvariables Sinussignal, auch als Sweep bezeichnet, generiert, welches mit Hilfe des Vibrators 2 in ein Schwingungssignal umgewandelt wird und in das Gelenk 9 eingeleitet wird. Das Kraftsignal Fo sowie das Beschleunigungssignal bo der zwei Sensoren 20, 21 wird dem Computer 26 zugeführt, wobei der Terzbandanalysator 26f eine Mittelwertbildung ausführt. Zur Erhöhung der Messdatenqualität können mit dem Terzbandanalysator 26f mehrere nacheinanderfolgende Messdurchgänge gemittelt werden, indem das durchlaufende Frequenzsignal mehrmals am Vibrator 2 angelegt wird. Das durchlaufende Frequenzsignal startet beispielsweise mit einer Frequenz von 1 Herz und erhöht daraufhin die Frequenz kontinuierlich bis 400 Hz. Dieser Vorgang wird zur Mittelwertbildung des Terzbandanalysators 26f mehrmals wiederholt, beispielsweise 10 mal. Nach Abschluss dieser Messung werden die gemittelten Terzbandspektren von der Signalverarbeitungsvorrichtung 26d gelesen und daraus für jede Terz ein diskreter mechanischer Impedanzwert berechnet. Fig. 5 zeigt den Verlauf der mechanischen Impedanz $Z/j\omega = F/b$ in Funktion der Frequenz $\omega$ wobei diese Impedanz basierend auf gemittelten Terzbandspektren berechnet wurde. Auf der Abszisse ist die Amplitude log A und die Phase dargestellt. Die Kurve 18 zeigt den Amplitudenverlauf und die Kurve 19 den Phasenverlauf der mechanischen Impedanz $Z/j\omega$. Auf der Abszisse sind im Abschnitt 17a und 17b die einzelnen Terzbänder, beginnend bei 1,25 Hz und endend mit 400 Hz dargestellt. Nachfolgend wird jeder gemessenen Impedanzkurve 18,19 der Zustand des Knorpelschadens zugeordnet, wobei der Zustand zum Beispiel während einer Operation bestimmt wird, welche nach der Messung durchgeführt wird. Zum Abschluss der Trainingsphase wird zwischen den mechanischen Impedanzen und den Zuständen des Knorpelschadens eine Diskriminanzanalyse durchgeführt und die relevanten Faktoren in der Signalauswertevorrichtung gespeichert.

Um nunmehr den Knorpelschaden eines Gelenkes zu erfassen wird, wie in Fig. 6 dargestellt, das zu untersuchende Gelenk 9 mit einer Schwingung angeregt, die Signale von der Signalverarbeitungsvorrichtung 26d gemessen und daraus die Impedanzkurve 18, 19 berechnet, worauf in der Signalauswertevorrichtung 26e die Impedanzkurve 18, 19 mit den relevanten Faktoren gewichtet wird und damit der Zustand des Gelenkes klassifiziert wird. In einer einfachen Ausführungsform kann dem Rechner 26 eine Ausgabevorrichtung 26b zugeordnet sein, welche den Zustand des Gelenkes 9 beispielsweise mit "gesund" oder "geschädigt" anzeigt.

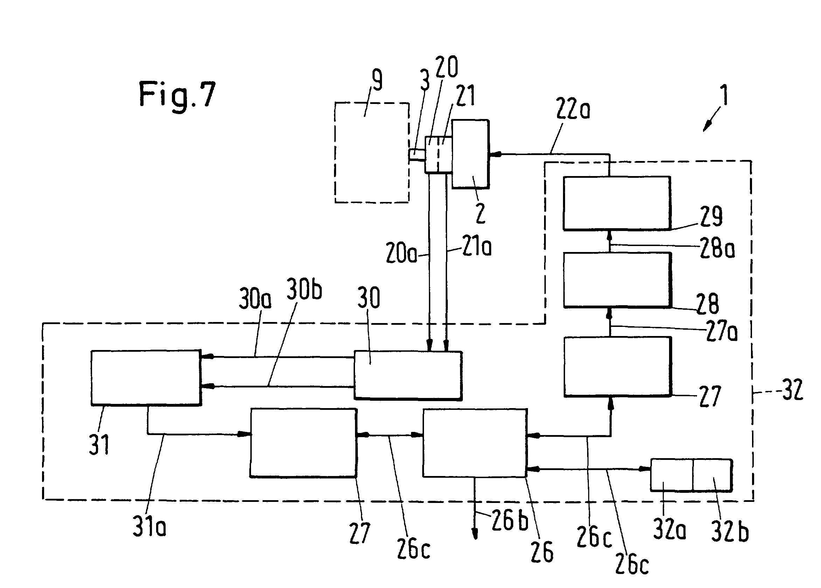

Fig. 7 zeigt den schematischen Aufbau eines weiteren Ausführungsbeispieles einer Vorrichtung 1 zum Erfassen eines Knorpelschadens, welche einen Vibrator 2 mit Schwingungsübertragungsmittel 3, Sensoren 20, 21 sowie ein tragbares Diagnosegerät 32 umfasst. Der Mikroprozessor 26 ist über einen Datenbus 26c mit einem Speicher 27 verbunden, welcher über eine elektrische Leitung 27a mit einem Digital-Analog-Wandler 28 verbunden ist. Der Ausgang des Wandlers 28 ist über eine elektrische Leitung 28a mit einem Verstärker 29, und dieser über eine elektrische Leitung 22a mit dem Vibrator 2 verbunden. Der Mikroprozessor 26 generiert ein digitales Ansteuersignal für den Vibrator 2, welches im Wandler 28 in ein analoges Wechselsignal umgewandelt wird und dem Vibrator 2 zugeleitet wird. Die Messsignale der Sensoren 20, 21 werden über elektrische Leitungen 20a, 21a einem Verstärker 30 und nachfolgend über elektrische Leitungen 30a, 30b einem Analog-Digital-Wandler 31 und nachfolgend als digitales Signal über die elektrische Leitung 31 a dem Speicher 27 zugeführt, auf welchen der Mikroprozessor über den Datenbus 26c Zugriff hat. Der Mikroprozessor 26 ist zudem über den Datenbus 26c mit einer Anzeigevorrichtung 32a sowie mit einer Eingabevorrichtung 32b verbunden.

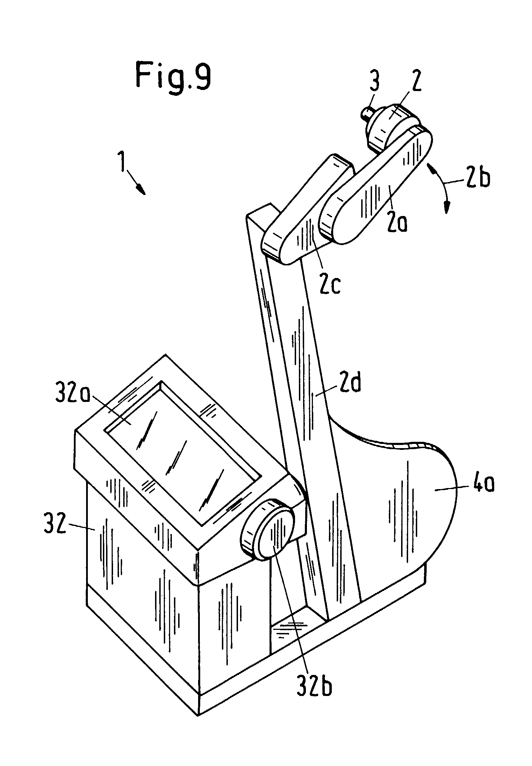

Fig. 9 zeigt ein weiteres Ausführungsbeispiel der Vorrichtung 1 zum Erfassen eines Knorpelschadens. Der Vibrator 2 mit Schwingungsübertragungsmittel 3 ist über die Halterungen 2a, 2c und 2d mit der Abstützvorrichtung 4a sowie mit der Diagnosevorrichtung 32 verbunden. Zum Messen des Knorpelschadens eines Kniegelenks 9 wird der Unterschenkel an die Abstützvorrichtung 4a angelehnt und der Vibrator 2 über eine Schwenkbewegung 2b der Halterung 2a derart bezüglich dem Knie angepasst angeordnet und fixiert, dass die Schwingung, wie in Fig. 1 dargestellt, über den Ankoppelungsbereich ME in das erste Gelenkteil 9a eingeleitet wird. Sobald die Messung durchgeführt ist, was abhängig von der Anzahl Messwiederholungen gesamthaft beispielsweise 10 bis 30 Sekunden dauert, wird die Beurteilung des Zustandes des Gelenkes auf der Anzeigevorrichtung 32a beispielsweise mit "gesund" oder "geschädigt" angezeigt. Es kann sich als vorteilhaft erweisen vor der Messung der Diagnosevorrichtung 32 noch Daten über die Eingabevorrichtung 32b oder eine berührungsempfindliche Anzeigevorrichtung 32a einzugeben, zum Beispiel das Alter oder das Gewicht der zu untersuchenden Person.

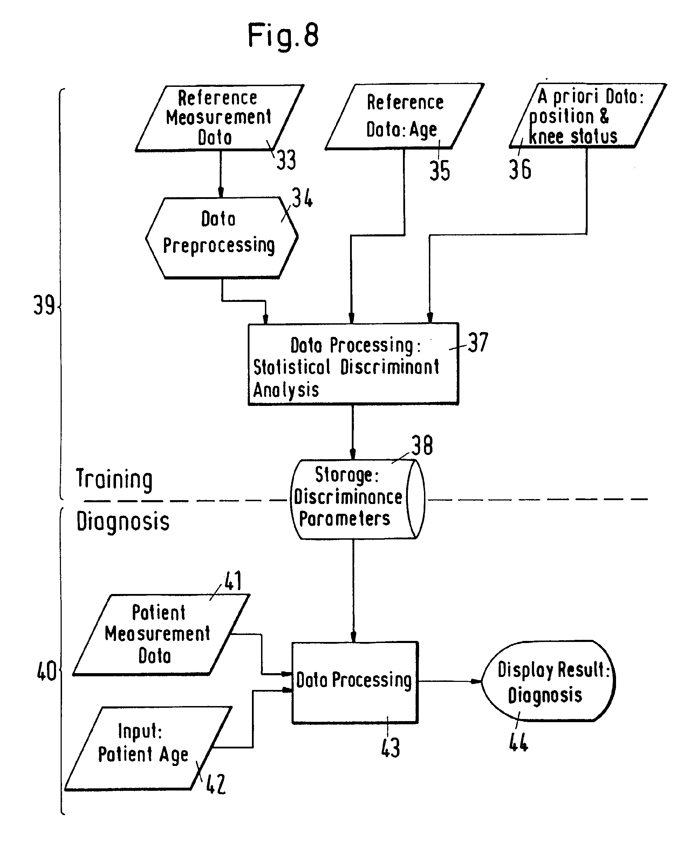

Fig. 8 zeigt ein Ablaufdiagramm zum erfassen des Knorpelschadens eines Gelenkes 9 während einer Trainings- oder Eichphase 39 und während einer Mess- bzw. Diagnosephase 40.

Während der Trainingsphase 39 werden, wie bereits beschrieben, das Schwingungsverhalten der Gelenke mehrerer Personen gemessen, und die Terzbandspektren der Kraft Fo und der Beschleunigung bo im Referenzdatensatz 33 abgelegt. Die Messdatenerfassung erfolgt vorzugsweise immer mit derselben Anordnung und mit derselben Schwingungseinleitung, beispielsweise immer mit der in Fig. 1 und Fig. 3a dargestellten Anordnung und Kniestellung. Zudem wird für jede Person das Alter im Referenzdatensatz 35 sowie die Stellung des Beines, das heisst gestrecktes oder angewinkeltes beziehungsweise belastetes oder unbelastetes Bein, sowie der Zustand des Knies im Referenzdatensatz 36 abgespeichert.

Nachdem genügend Referenzdaten erfasst sind, wird zwischen den mechanischen Impedanzen und den Zuständen der Gelenke ein mathematischer Zusammenhang ermittelt. Im dargestellten Ausführungsbeispiel wird dazu eine Diskriminanzanalyse durchgeführt. Dazu wird ausgehend von den Terzbandspektren der Kraft Fo sowie der Beschleunigung bo in einem Rechenverfahren 34 die mechanische Impedanz berechnet. Das Rechenverfahren 34 kann beispielsweise unter Verwendung des Standardsoftwarepaketes MATLAB® erfolgen. Der mathematische Zusammenhang könnte beispielsweise auch mit Hilfe einer der folgenden mathematischen Methoden ermittelt werden:

- classification trees,
- neuronales Netzwerk,
- fuzzy logic.

Nachfolgend wird mit den Referenzdaten aller gemessenen Personen im Verfahrensschritt 37 eine Diskriminanzanalyse durchgeführt. In der Diskriminanzanalyse wird beispielsweise zwischen jüngeren und älteren Personen sowie zwischen einem unbelasteten und einem belasteten Gelenk unterschieden. Die Diskriminanzanalyse 37 kann beispielsweise unter Verwendung des Standardsoftwarepaketes STATISTICA (StatSoft Inc. (1995). STATISTICA for Windows. Tuls, OK: StatSoft, Inc., 2300 East 14th Street, Tulsa, OK, 74104-4442) erfolgen. Die ermittelten Parameter werden in der Datenbank 38 abgelegt.

Während der Diagnosephase 40 wird in das zu untersuchende Gelenk 9 eine Schwingung eingeleitet und die mechanische Impedanz gemessen, wobei die Schwingungseinleitung sowie die Anordnung des Gelenkes möglichst wie während der Erfassung der Referenzdaten zu erfolgen hat. Zudem wird das Alter der untersuchten Person und ev. weitere Daten, z.B. die Belastung des Gelenkes 9, erfasst. Nach erfolgter Messung wird die mechanische Impedanz im Verfahrensschritt 43 aus den Werten Fo und bo berechnet, und anschliessend die mechanische Impedanz mit dem in der Datenbank 38 gespeicherten Werten gewichtet und dadurch der Zustand des Gelenkes 9 berechnet. Dieser Wert wird an einem Bildschirm 44 angezeigt.

**Patentansprüche**

1.  Vorrichtung (1) zum Erfassen des Zustandes, insbesondere des Knorpelzustandes oder des Meniskuszustandes, eines sich innerhalb eines Körpers (5) befindlichen Gelenkes (9) umfassend:

    - eine Schwingungserzeugungsvorrichtung (2) zum Erzeugen tieffrequenter Schwingungen, insbesondere im Bereich zwischen 1 und 500 Hz,
    - ein Schwingungsübertragungsmittel (3) zum Einleiten der Schwingungen in den Körper (5),
    - zumindest einen Sensor (20, 21) zum Messen einer Systemschwingung,
    - eine Signalverarbeitungsvorrichtung (26d), welche zumindest mit dem einen Sensor (20, 21) verbunden ist und aus dem Sensorsignal Werte der mechanischen Impedanz bei zumindest zwei unterschiedlichen Frequenzen ermittelt,
    - eine Signalauswertevorrichtung (26e), welche aus der ermittelten mechanischen Impedanz und vorherbestimmten Parametern mit Hilfe einer Ergebnisfunktion eine Ergebnisgrösse ermittelt, welche den Zustand des Gelenkes (9) bewertet.

2. Vorrichtung nach Anspruch 1, dadurch gekennzeichnet, dass die Schwingungserzeugungsvorrichtung (2) als eine im wesentlichen ideale Kraftquelle oder eine im wesentlichen ideale Verschiebungsquelle ausgestaltet ist.

3. Vorrichtung nach Anspruch 1, dadurch gekennzeichnet, dass ein erster und ein zweiter Sensor (20, 21) zum Messen je einer Systemschwingung vorgesehen ist, und dass die Signalverarbeitungsvorrichtung (26d) mit den beiden Sensoren (20, 21) verbunden ist und aus den beiden Sensorsignalen die mechanische Impedanz zu ermitteln erlaubt.

4. Vorrichtung nach Anspruch 3, dadurch gekennzeichnet, dass der erste Sensor (20) als ein Kraftsensor und der zweite Sensor (21) als ein Beschleunigungs- oder Geschwindigkeitssensor ausgebildet ist.

5. Vorrichtung nach einem der vorhergehenden Ansprüche, dadurch gekennzeichnet, dass die Schwingungserzeugungsvorrichtung (2) als ein Vibrator ausgestaltet ist, welcher von einem Signalgenerator (22) angesteuert ist.

6. Vorrichtung nach Anspruch 3, dadurch gekennzeichnet, dass die Schwingungserzeugungsvorrichtung (2) als ein Hammer ausgestaltet ist.

7. Vorrichtung nach einem der vorhergehenden Ansprüche, dadurch gekennzeichnet, dass der erste und der zweite Sensor (20, 21) im Schwingungsübertragungsmittel (3) angeordnet sind.

8. Vorrichtung nach einem der vorhergehenden Ansprüche gekennzeichnet durch eine Halte- oder Einspannvorrichtung (4) zum Fixieren eines Körperteils (5).

9. Vorrichtung nach einem der vorhergehenden Ansprüche, dadurch gekennzeichnet, dass die Signalverarbeitungsvorrichtung (26d) eine Vorrichtung zur Frequenzanalyse und zur Mittelwertbildung der Sensorsignale, insbesondere zur Mittelwertbildung in Terzen umfasst.

10. Vorrichtung nach einem der Ansprüche 3 bis 7, dadurch gekennzeichnet, dass der Vibrator (2) eine Federsteifigkeit zwischen 50 N/cm und 150 N/cm aufweist.

11. Vorrichtung nach einem der vorhergehenden Ansprüche, dadurch gekennzeichnet, dass die Schwingungserzeugungsvorrichtung (2) als elektrodynamischer, nach dem Induktionsprinzip arbeitender Wandler ausgestaltet ist.

12. Verfahren zum Erfassen des Zustandes, insbesondere des Knorpel- oder Meniskuszustandes, eines sich innerhalb des Körpers befindlichen Gelenkes, dadurch gekennzeichnet,

   - dass ein Schwingungsanregungssignal an einer Stelle in den Körper eingeleitet wird,
   - dass mittels zumindest einem Sensor eine Systemschwingung gemessen und daraus Werte der mechanischen Impedanz bei zumindest zwei unterschiedlichen Frequenzen ermittelt werden,
   - dass ausgehend von der ermittelten mechanischen Impedanz mit vorherbestimmten Parametern und mit Hilfe einer Ergebnisfunktion eine Ergebnisgrösse ermittelt wird,
   - und dass die Ergebnisgrösse als Angabe über den Zustand des Gelenkes verwendet wird.

13. Verfahren nach Anspruch 12, dadurch gekennzeichnet, dass das Schwingungsanregungssignal mittels einer Kraftquelle oder einer Verschiebungsquelle in den Körper eingeleitet wird, dass mittels einem Sensor eine Systemschwingung gemessen wird, und dass aus dem Schwingungsanregungssignal sowie der gemessenen Systemschwingung eine der mechanischen Impedanz ähnliche Grösse ermittelt wird.

14. Verfahren nach Anspruch 12 oder 13, dadurch gekennzeichnet, dass mittels zweier Sensoren je eine Systemschwingung gemessen und daraus die mechanische Impedanz ermittelt wird.

15. Verfahren nach einem der Ansprüche 12 bis 14, dadurch gekennzeichnet, dass das Schwingungsanregungssignal nach Möglichkeit in einen Knochen eingeleitet wird, und dass der Einleitungsort des Schwingungssignals derart am Körper gewählt wird, dass sich zwischen Einleitungsort und Knochen möglichst wenig anderes Gewebe befindet.

16. Verfahren nach einem der Ansprüche 12 bis 15, dadurch gekennzeichnet, dass das Schwingungsanregungssignal im Bereich der Patella, der Epicondylus medialis oder lateralis, der Condylus medialis oder lateralis, der Tuberositas tibiae oder der Tibiofibularis eingeleitet wird.

17. Verfahren nach einem der Ansprüche 12 bis 16, dadurch gekennzeichnet, dass als Schwingungsanregungssignal ein Hammerschlag oder ein Frequenz variierendes Sinussignal oder ein Rauschen verwendet wird.

18. Verfahren nach einem der Ansprüche 12 bis 17, dadurch gekennzeichnet, dass ein Teil des Körpers nahe des Gelenks eingespannt wird, oder dass an einem Teil des Körpers ein zusätzliches mechanisches System wie eine Masse, eine Dämpfung oder eine Feder angekoppelt wird.

19. Verfahren zum Erfassen eines Knorpelschadens eines sich innerhalb des Körpers befindlichen Gelenkes, dadurch gekennzeichnet,
dass in einem ersten Verfahrensschritt eine Trainingsphase durchgeführt wird,

-   indem eine Mehrzahl von Gelenken unterschiedlicher Subjekte einem Schwingungsanregungssignal ausgesetzt wird, die resultierende Körperschwingung gemessen und daraus die mechanische Impedanz ermittelt wird,
-   indem der Zustand der Gelenke klassifiziert wird,
-   und indem zwischen den mechanischen Impedanzen und den Zuständen der Gelenke ein mathematischer Zusammenhang ermittelt wird,

und dass in einem zweiten Verfahrensschritt eine Bewertung durchgeführt wird,

-   indem ein zu untersuchendes Gelenk dem Schwingungsanregungssignal ausgesetzt wird,
-   indem die resultierende Systemschwingung gemessen und daraus die mechanische Impedanz ermittelt wird,
-   und indem aus der mechanischen Impedanz mit Hilfe des ermittelten mathematischen Zusammenhangs der Zustand des Gelenkes bewertet wird.

# Fig.1

# Fig.2

Fig.3a

Fo   12      9
bo        9a
          9f
    4        4
        9e

Fig.3b

Fo   12   9a   9
bo        9f
          4
     9e

Fig.3c

Fo   12   9a
bo        9f
          F1   4
     9e

Fig.3d

Fo        9a
bo        9f
          9e   b1

Fig.4

Fig.5

EP 1 095 615 A1

# Fig. 6

Fig.7

EP 1 095 615 A1

# Fig.8

Fig.9

**Europäisches**
**Patentamt**

**EUROPÄISCHER RECHERCHENBERICHT**

Nummer der Anmeldung

EP 00 81 0903

| | **EINSCHLÄGIGE DOKUMENTE** | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
| A | EP 0 764 842 A (OMATA SADAO) 26. März 1997 (1997-03-26) * Spalte 10, Zeile 30 – Spalte 11, Zeile 17 * * Spalte 18, Zeile 1 – Spalte 20, Zeile 49 * * Spalte 24, Zeile 21 – Spalte 25, Zeile 56 * * Spalte 40, Zeile 57 – Spalte 41, Zeile 21 * --- | 1,12,19 | A61B5/103 A61B9/00 |
| A | US 4 986 281 A (PREVES DAVID A ET AL) 22. Januar 1991 (1991-01-22) * Spalte 1, Zeile 22 – Zeile 64 * ----- | 1,12,19 | |
| | | | RECHERCHIERTE SACHGEBIETE (Int.Cl.7) A61B |

Der vorliegende Recherchenbericht wurde für alle Patentansprüche erstellt

| Recherchenort | Abschlußdatum der Recherche | Prüfer |
|---|---|---|
| DEN HAAG | 24. Januar 2001 | Martelli, L |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 00 81 0903

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

24-01-2001

| Im Recherchenbericht angeführtes Patentdokument | Datum der Veröffentlichung | Mitglied(er) der Patentfamilie | Datum der Veröffentlichung |
|---|---|---|---|
| EP 0764842 A | 26-03-1997 | JP 9145691 A<br>AU 709813 B<br>AU 6564996 A<br>US 5766137 A | 06-06-1997<br>09-09-1999<br>27-03-1997<br>16-06-1998 |
| US 4986281 A | 22-01-1991 | KEINE | |

EPO FORM P0461

Für nähere Einzelheiten zu diesem Anhang : siehe Amtsblatt des Europäischen Patentamts, Nr.12/82